# EUROPEAN PATENT SPECIFICATION

(11) **EP 2 478 152 B1**
(45) Date of publication and mention of the grant of the patent: **31.12.2014**
(21) Application number: 10754929.7
(22) Date of filing: 17.09.2010
(51) Int. Cl.: D21H 21/30, C07D 251/68

(54) **DISULFO-TYPE FLUORESCENT WHITENING AGENTS IN COATING APPLICATIONS**
DISULFO-FLUORESZENTE AUFHELLER BEI BESCHICHTUNGSANWENDUNGEN
AGENTS DE BLANCHIMENT FLUORESCENTS DE TYPE DISULFO DANS DES APPLICATIONS DE REVÊTEMENT

(30) Priority: 17.09.2009 EP 09170579
(43) Date of publication of application: 25.07.2012
(73) Proprietor: Blankophor GmbH & Co. KG, 49577 Ankum (DE)
(72) Inventor: HUNKE, Bernhard, 53773 Hennef (DE); KRAEMER, Michael, 51515 Kürten (DE); TAUBER, Andrei, 50735 Köln (DE); KLUG, Günter, 40764 Langenfeld (DE)
(74) Representative: Bublak, Wolfgang
(86) International application number: PCT/EP2010/063705
(87) International publication number: WO 2011/033066

(56) References cited:
- EP-A- 0 884 312
- EP-A- 1 752 453
- WO-A-01/19804
- WO-A-02/055646
- WO-A1-98/42685
- WO-A1-03/078724

## Description

The present invention relates to the use of specific disulfo-type fluorescent whitening agents for optically brightening or preparing coating slips, coating slips per se and their use for the production of brightened papers.

It is well known that the whiteness of paper and board can be improved by the addition of fluorescent whitening agents. The most important fluorescent whitening agents used in the paper and board industry are anilino-substituted bistriazinyl derivatives of 4,4'-diaminostilbene-2,2'-disulfonic acid (flavonic acid). From these fluorescent whitening agents disulfo-, tetrasulfo- and hexasulfo-types are known. The disulfo-type fluorescent whitening agents with no sulfonic acid groups at the aniline rings have a low solubility in water and a high affinity for cellulose fibres. They are especially suitable for use at the wet-end of paper making process. The hexasulfo-type fluorescent whitening agents with two sulfonic acid groups at each aniline ring have a high solubility in water and a low affinity for cellulose fibres. They are more specialty products when very high whiteness is desired. The tetrasulfo-type fluorescent whitening agents with one sulfonic acid group at each aniline ring exhibit a behaviour between the disulfo- and hexasulfo-type fluorescent whitening agents and are most commonly used for whitening paper or board.

The brightening of uncoated papers or untreated coating papers can be effected by use in the pulp and/or surface application of fluorescent whitening agents, which are usually present for this purpose in dissolved form. In the production of coated papers, the addition of fluorescent whitening agents to the coating slip is customary, so that, in the finished coated paper, the fluorescent whitening agents is also present in the pigment layer applied to the paper. Coated papers are particularly suitable for the production of high-quality prints. There is a progressive trend towards coated papers having high whiteness and therefore a demand for fluorescent whitening agents which are as effective as possible as coating slip components.

The common disulfo-type fluorescent whitening agents with no sulfonic acid groups at the aniline rings have low solubility in water which causes problems in concentrated liquid formulations, e.g. precipitates are formed, and thus require solubilising auxiliaries, such as urea, triethanolamine or diethylene glycol, which, however, contaminate the effluent from the paper mill after repulping coated broke and thus are undesired.

EP-A-1 355 004 describes tetrasulfo-type fluorescent whitening agents for the brightening of coating slips. WO 2006/045714 A1 discloses compositions containing fluorescent whitening agents, a polymer formed from an ethylenically unsaturated monomer or monomer blend, and, optionally, polyethylene glycol, for coating, size press or film press applications. EP-A-1 752 453 teaches storage stable solutions of disulfo-type fluorescent whitening agents which contain specific counter-ions for the sulfonic acid groups, which counter-ions are derived from specific aminoalkanols. WO 02/055646 A1 discloses concentrated aqueous solutions containing a mixture of two specific disulfo-type fluorescent whitening agents. Further, slurries or dispersions of disulfo-type fluorescent whitening agents in water are known from EP-A- 0 884 312.

WO 01/19804 A1 is directed to specific 4,4'-diaminostilbene-2,2'-disulfonic acid compounds being useful as fluorescent whitening agents or for removing stain in photographic materials. WO 98/42685 A1 refers to other specific 4,4'-diaminostilbene-2,2'-disulfonic acid compounds being useful as fluorescent whitening agents or for inhibiting (quenching) the effect of anionic fluorescent whitening agents on substrates. WO 03/078724 A1 discloses a process for improving the sun protection factor of cellulosic fiber materials, which comprises contacting said materials with defined 4,4'-diaminostilbene-2,2'-disulfonic acid compounds.

Surprisingly, it has been found that problems of the prior art can be overcome by using specific disulfo-type fluorescent whitening agents having carboxylic acid groups at the aniline rings in coating slips. These disulfo-type fluorescent whitening agents have higher solubility in water than the commonly used disulfo-type fluorescent whitening agents with no sulfonic acid groups at the aniline rings. Additionally, it was found that these specific disulfo-type fluorescent whitening agents have significantly better whitening performance in coating slips.

Therefore, the present invention relates to the use of at least one fluorescent whitening agent of formula (I) 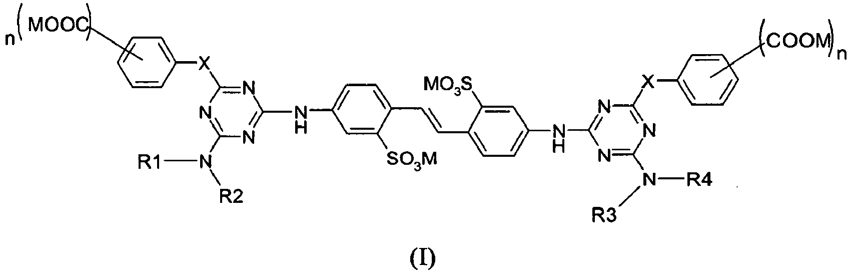 wherein
X represents, independently of each other, O or NR', where R' is hydrogen or C₁-C₃ alkyl;
n is 1 or 2;
R₁, R₂, R₃ and R₄ represent, independently of each other, hydrogen, cyano, C₁-C₄ alkyl, C₂ - C₄ cyanoalkyl, C₂ - C₄ hydroxyalkyl, or C₁ - C₄ alkoxyalkyl, wherein alkyl is linear or branched; or R₁ and R₂ or R₃ and R₄ independently of each other together with N atom form morpholine, piperidine or pyrrolidine ring; or -(CH₂)₁-SO₃M, where 1 is 1, 2 or 3; or -(CH₂)ᵢ-COOR, -(CH₂)i-CONHR, -(CH₂)ᵢ-OR, where i is an integer from 1 to 4, R is C₁-C₃ alkyl or has the same meaning as M; M represents hydrogen, or one equivalent of a cation, in particular Li, Na, K, Ca, Mg, ammonium, or ammonium which is mono-, di-, tri- or tetra-substituted by C₁ - C₄ alkyl or C₂ - C₄ hydroxyalkyl;
for the whitening (brightening) of coating slips containing at least one synthetic binder and at least one synthetic co-binder differing from the synthetic binder.

The invention also relates to coating slips containing at least one fluorescent whitening agent of formula (I), and their use for preparing coated papers, and the paper obtainable by a process using said coating slip. In addition, the invention relates to a preparation containing at least one fluorescent whitening agent of formula (I) and polyethylene glycol. Preferred embodiments of the invention are described in the description hereinafter, the figures and the claims.
Fig. 1 is a diagram showing the whitening performance of different fluorescent whitening agents in coating slips.
Fig. 2 is a further diagram showing the whitening performance of different fluorescent whitening agents in coating slips.
Fig. 3 is another diagram showing the whitening performance of different fluorescent whitening agents in coating slips.
Fig. 4 is a diagram showing the whitening performance of different fluorescent whitening agents with and without polyethylene glycol in coating slips.

According to the invention at least one fluorescent whitening agent of the above defined formula (I) is used. In a preferred embodiment, X represents NR' in formula (I). In another preferred embodiment, n is 1. In the context of the invention, in formula (I) the alkyl group can be linear or branched, and the possible substituents of the alkyl group, which are alkoxy, cyano, and/or hydroxyl groups, can be attached at any position of the alkyl chain. In the present invention, C₁ - C₄ alkoxyalkyl means C₁ - C₄ alkyl substituted with C₁ - C₄ alkoxy. In a preferred embodiment, R₁, R₂, R₃ and R₄ represent, independently of each other, C₂ - C₄ hydroxyalkyl, C₁ - C₄ alkoxyalkyl or C₁ - C₄ alkyl, preferably C₂ - C₄ hydroxyalkyl or C₁ - C₄ alkoxyalkyl, in particular hydroxyethyl or hydroxyisopropyl. Most preferably, R₁, R₂, R₃ and R₄ represent hydroxyethyl or hydroxyisopropyl. The carboxy groups at the aniline rings can be in *ortho*-, *meta-* or *para-*position, preferably they are in *ortho-* or *para*-position, in particular in *para*-position.

Preferred embodiments of M are hydrogen, Na, K, Ca, Mg, in particular M is Na, K or hydrogen, most preferred is Na.

Preferred fluorescent whitening agents are the fluorescent whitening agents of the following formula (Ia) and formula (Ib), wherein the carboxylic acid residues are, independently of each other, in *ortho-* or *para*-position, preferably in *para-*position:

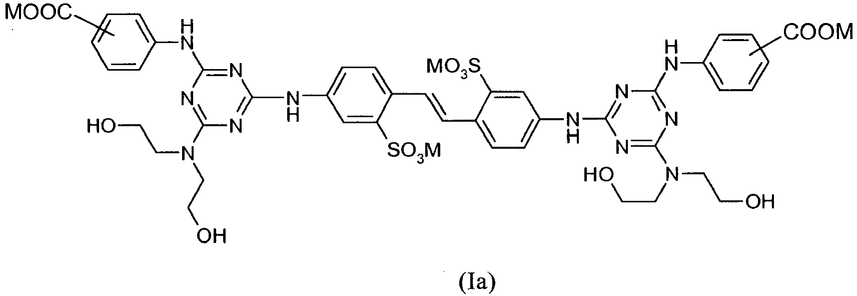

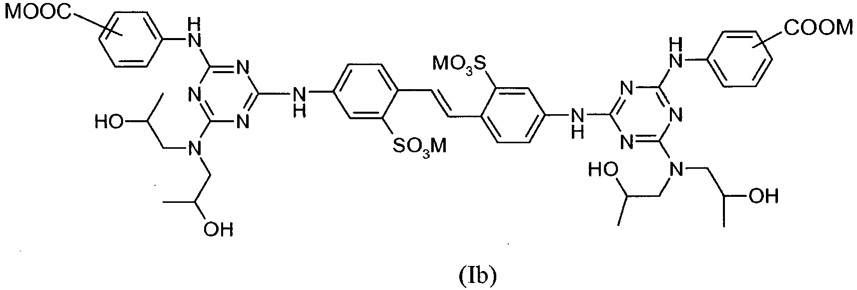

The fluorescent whitening agents of formula (I) can be prepared by known procedures, and are used as free acids or as salts thereof, preferably alkali metal salts. Generally, the compounds are prepared by reacting cyanuric chloride with 4,4'-diaminostilbene-2,2'-disulfonic acid or a salt thereof, an appropriate carboxyl acid group-containing derivative, e.g. 2- or 4-aminobenzoic acid, and substituted aliphatic amines or heterocyclic compounds. PL patent 61710 discloses the preparation of some specific fluorescent whitening agents of the above defined formula (I) with one carboxylic acid group in p-position of each aniline ring. GDR (DDR) patent 55 668 discloses a further process for preparing some specific fluorescent whitening agents of the above defined formula (I) with one or two carboxylic acid groups at each aniline ring. The purification of the fluorescent whitening agents of formula (I) is easier and thus more cost-effective than for commonly used disulfo-type fluorescent whitening agents, since isolation steps can be avoided. The purification could be carried out by, for example, membrane filtration. In contrast to the water evaporation or salt precipitation steps disclosed in PL patent 61710, the purification of the fluorescent whitening agents of formula (I) can be achieved by membrane filtration and the product obtained can be used as such. This is due to the surprisingly higher solubility of fluorescent whitening agent of formula (I).

One or more fluorescent whitening agents of formula (I) are used for preparation or brightening of the coating slips. In a preferred embodiment, one fluorescent whitening agent of the formula (I) is used. In another preferred embodiment, two or three fluorescent whitening agents of the formula (I) are used. It is also possible that other known fluorescent whitening agents are additionally used.

In a preferred embodiment, the fluorescent whitening agents are used in form of aqueous preparations.

Aqueous preparations can be prepared from crude solutions, from concentrated and desalinated solutions or from water-containing press cakes. To build up particularly good whiteness, it is advantageous to incorporate so-called carrier substances into the aqueous fluorescent whitening agent preparations.

The aqueous fluorescent whitening agent preparations preferably contain
a) from 10 to 40% by weight of at least one fluorescent whitening agent of formula (I),
b) from 0 to 30% by weight of standardizing agent,
c) from 0 to 2% by weight of inorganic salts, and
d) from 28 to 90% by weight of water,
in each case based on 100% by weight of the preparation.

Customary standardizing agents are, for example, urea, diethylene glycol, triethylene glycol, propanediol, glycerol, ε-caprolactam, ethanolamine, diethanolamine and triethanolamine. In each case, preparations free of standardizing agents are preferred. The inorganic salts are inorganic salts coming from the production process.

In another preferred embodiment, the aqueous fluorescent whitening agent preparations contain:
a) from 5 to 40% by weight of at least one fluorescent whitening agent of the formula (I),
b) from 1 to 50% by weight of at least one carrier substance,
c) from 0 to 2% by weight of inorganic salts, and
d) from 8 to 94% by weight of water,
in each case based on 100% by weight of the preparation.

Suitable carrier substances are in general hydrophilic polymers having the ability to form hydrogen bridge bonds. Preferred carrier substances are polyvinyl alcohols, carboxymethylcelluloses, polyethylene glycols, or mixtures of these substances, it being possible for these polymers optionally to be modified. Preferred polyvinyl alcohols are those having a degree of hydrolysis of >85%. Preferred carboxymethylcelluloses are those having a degree of substitution DS of >0.5. Preferred polyethylene glycols are those having a number average molecular weight Mn of from 200 to 8,000 g/mol, preferably from 800 to 4,000 g/mol. Suitable carriers are further, for example, natural, derivatized or degraded starches, alginates, casein, proteins, polyacrylamides, polyacrylic acids, hydroxyalkylcellulose, and polyvinylpyrrolidone. Most preferably, polyethylene glycols are used as carrier.

In addition, both carrier-free and carrier-containing preparations may contain small amounts, usually amounts of less than 5% by weight, of further auxiliaries, such as dispersants, thickeners, antifreezes, preservatives, complexing agents, etc., or organic and inorganic byproducts from the fluorescent whitening agent synthesis which were not completely removed during the working up.

The carrier-containing preparations may additionally contain standardizing agents for increasing the solubility and shelf life.

The carrier-free aqueous fluorescent whitening agent preparations can be prepared in general by adjusting a fluorescent whitening agent solution (crude or membrane-filtered) with a base to a neutral to weakly alkaline pH value, optionally adding and dissolving one ore more standardizing agents, and optionally diluting with water to the desired final concentration. If the fluorescent whitening agent is used in the form of a water-moist press cake or dry powder, a certain amount of the press cake or powder is completely dissolved in water with addition of base and with stirring and optionally at elevated temperatures, and optionally adjusted to the desired concentration by further addition of water.

Preferred bases for this purpose are alkali metal hydroxides, demineralised water being preferred for dilution. The pH value established is preferably in the range of from 7 to 11, preferably from 8 to 10. Temperatures of from 25 to 80°C are customary for the dissolution.

The carrier-containing preparations can be prepared in general in an analogous manner, the carrier substance also being added at any desired time during the preparation process. If the carrier substance is added in solid form, it is generally completely dissolved with stirring and optionally at elevated temperatures, so that a homogeneous liquid preparation forms. The viscosity of the carrier-containing preparations at room temperature is preferably less than 3,000 mPas. The customary dissolution temperature is in the range from 25 to 100°C.

Concentrated, aqueous fluorescent whitening agent preparations are usually characterized by the so-called E1/1 value. For this purpose, the extinction of a highly dilute solution of the preparation is determined by the customary UV/VIS spectroscopy methods known to a person skilled in the art, in a 1 cm cell at a certain wavelength. This wavelength corresponds to the long-wave absorption maximum of the respective fluorescent whitening agent molecule. In case of flavonate fluorescent whitening agents, it is about 350 mm. The E1/1 value then corresponds to the imaginary extinction value estimated for a 1% strength solution.

The E1/1 values of the fluorescent whitening agent used according to the invention are preferably from 50 to 180, particularly preferably from 70 to 140.

The coating slips to be brightened according to the invention contain at least one synthetic binder, in particular latex binder or polyacrylates, and at least one synthetic co-binder differing from the synthetic binder.

Suitable synthetic binders are, for example, latices based on styrene/butadiene, styrene/acrylate, or vinyl acetate. These polymers can optionally be modified by further monomers, such as acrylonitrile, acrylamide, α,β-unsaturated carboxylic acids, such as acrylic acid, methacrylic acid, itaconic acid or maleic acid, acrylates, vinyl esters, ethylene, vinyl chloride, vinylidene chloride, etc. In general, all customary synthetic binders, in particular latex binders and polyacrylates, which are used in the preparation of paper coating slips are suitable. Preferred latex binders are those based on styrene/butadiene.

Suitable synthetic co-binders differing from the synthetic binders are, for example, carboxymethylcellulose, hydroxyalkylcellulose, polyvinyl alcohol, and/or acrylate-based synthetic thickeners. Further suitable co-binders are the same compounds as those described above for the carriers. Preferred synthetic co-binders are polyvinyl alcohols, in particular those having a degree of hydrolysis of >85%, and in particular a Brookfield viscosity of 2-80 mPas (measured on a 4% strength aqueous solution at 20°C), carboxymethylcelluloses, in particular those having a degree of substitution of >0.5, and in particular a Brookfield viscosity of from 5 to 5000 mPas (measured on a 2% strength aqueous solution at 25°C) and mixtures of these two substances. The most preferred co-binders are polyvinyl alcohol, carboxymethylcellulose, or a mixture thereof

The coating slips to be brightened according to the invention preferably furthermore contain white pigments. Commonly used white pigments are calcium carbonate in natural or precipitated form, kaolin, talc, titanium dioxide, satin white, aluminium hydroxide, and barium sulfate, often also in form of mixtures thereof.

The coating slips to be brightened according to the invention may contain dispersants as optional further ingredients. Polyacrylates, polyphosphates and Na citrate are commonly used for that purpose. Further, polyaspartic acid is also suitable. Further optional additives are crosslinking agents. Examples of these are urea/formaldehyde resins, melamine/formaldehyde resins, glyoxal and ammonium/zirconium carbonate. Further, wet strength agents based on polyamidoamine/epichlorohydrin resins, glyoxalated polyacrylamides or hydrophilized polyisocyanates, as described, for example, in EP-A-825 181, are also suitable as crosslinking agents. Furthermore, antifoams, biocides, complexing agents, bases for pH adjustment, Ca stearate, optical brighteners other than those of the formula (I) and shading dyes may be used as further optional additives. Sometimes surface sizes are also added for imparting water repellency to the coating slip. Examples of these are polymer solutions based on styrene/acrylic acid, styrene/maleic anhydride or oligourethanes, and polymer dispersions based on acrylonitrile/acrylate or styrene/acrylate. The latter are described, for example in WO-A-99/42490.

The coating slips to be brightened according to the invention contain the synthetic binder preferably in an amount of from 2 to 20% by weight, in particular from 3 to 15 % by weight, and the synthetic co-binder in an amount of from 0.1 to 3% by weight, in particular from 0.15 to 2 % by weight, based in each case on 100% by weight of the white pigment of the coating slip.

The invention furthermore relates to a coating slip, in particular an aqueous coating slip or aqueous pigment preparation, comprising
at least one white pigment,
at least one synthetic binder,
at least one synthetic co-binder differing from the synthetic binder, and
at least one fluorescent whitening agent of the formula (I).

Preferably, the amount of synthetic binder (calculated as dry substance) is from 2 to 20% by weight, in particular from 3 to 15% by weight, and independently thereof the amount of co-binder is preferably from 0.1 to 3% by weight, in particular from 0.15 to 2% by weight. Preferably, the amount of fluorescent whitening agent of the formula (I) is from 0.025 to 1% by weight, in particular from 0.03 to 0.75% by weight, based in each case on the amount of 100% by weight of white pigment.

The preferred embodiments for white pigment, synthetic binder, synthetic co-binder, fluorescent whitening agent, and other additives are the same as described above.

The coating slip preferably additionally contains at least one dispersant, in particular in an amount of from 0.05 to 1% by weight, based on 100% by weight of the white pigment in the coating slip. Suitable dispersants are preferably polyacrylic acid and corresponding salts. The water content of the coating slip is preferably from 20 to 50% by weight, in particular from 25 to 45% by weight, based on the total amount of coating slip.

The invention furthermore relates to the use of the coating slips according to the invention for the production of coated papers.

The coating slips can preferably be applied to the paper once or several times by all application methods suitable for this purpose, such as by knife coating in various embodiments, air brush, blade, roll-coater, film press, casting methods, etc. The immobilization and drying of the coating slip is usually effected initially by contactless hot-air and/or IR drying, commonly followed by contact drying by means of heated rolls. Calendering for compaction, smoothing or influencing the gloss of the coated paper, for example by means of a calendar, is then usually carried out.

Suitable uncoated base papers or untreated coating papers, boards and cardboards are papers, boards and cardboards produced from bleached or unbleached, wood-containing or wood-free, waste paper-containing and deinked fibers. These may furthermore contain mineral filers, such as natural or precipitated chalk, kaolin, talc or annalines. The uncoated papers, boards and cardboards can be engine sized and/or surface sized, with the result that, inter alia, the penetration and the adhesion of the coating slip are influenced. Commonly used engine sizes are alkylke-tene dimers (AKD), alkenylsuccinic anhydride (ASA) and a combination of rosin size and alum, and commonly used surface sizes are the abovementioned polymer solutions based on styrene/acrylic acid, styrene/maleic anhydride or oligourethanes, and polymer dispersions based on acrylonitrile/acrylate or styrene/acrylate. For controlling the desired whiteness properties of the resulting coated paper, the base papers can be brightened in the pulp and/or surface brightened, for which purpose, for example, flavonate brighteners are used.

The invention further relates to the use of the above-described coating slips for whitening paper or producing coated papers, and further the papers obtained thereby. In addition, the present invention relates to a method for brightening a coating slip, in particular an aqueous coating slip, comprising at least one synthetic binder and at least one synthetic co-binder differing from the synthetic binder, which method comprises treating the coating slip with a fluorescent whitening agent composition comprising a fluorescent whitening agent of formula (I) as described above. Likewise, the synthetic binder, the synthetic co-binder, and the other optional additives are the same as described above.

In another embodiment, the invention relates to a preparation comprising at least one disulfo-type fluorescent whitening agent of the formula (I) and polyethylene glycol. The fluorescent whitening agent and polyethylene glycol are the same as defined above. The preparation is preferably present in liquid form. It can be prepared from fluorescent whitening agent, water, and polyethylene glycol, and further optionally some amount of base for pH adjustment. The preparation can be used for addition to or preparation of a coating slip.

The whiteness of the papers produced can be characterized by the CIE whiteness. Different fluorescent whitening agents can be compared to each other with respect to the saturation behaviour when determined according to CIE whiteness. In other words, if a larger amount of fluorescent whitening agent is used and no further increase in whiteness is found, there is saturation behaviour and there may even be adverse effects on the whiteness when using higher amounts. The effect of saturation is also referred to as greening. The greening limit, i.e. the point at which increasing amounts of fluorescent whitening agent used results in virtually no further increase in whiteness, can be derived, for example, from the a*-b* diagram, where a* and b* are the colour coordinates in the CIE-L*a*b* system.

The following examples illustrate the invention and show preferred embodiments without limiting the scope of protection.

### EXAMPLES

The whitening performance of different fluorescent whitening agents in coating slip applications was studied using the following procedure.

First, fluorescent whitening agent preparations were prepared as follows. Presscakes of the fluorescent whitening agents were dissolved in demineralized water at 80°C together with caustic soda at pH 9 to a concentration of 2.8%. Fluorescent whitening agent preparations containing polyethylene glycol were prepared as follows. Presscakes of the fluorescent whitening agents were dissolved in demineralized water at 80°C together with caustic soda at pH 9. Then, polyethylene glycol, namely PEG 1550, was added. The resulting concentration of fluorescent whitening agent was 2.8% and that of PEG 1550 was 4.5%. The preparations were handled at a temperature between 20-50°C.

A paper coating slip was prepared from the following components:
- 100 parts of white pigment (chalk/kaolin mixture),
- 10 parts of Litex P 7110 as a binder, calculated as dry substance (styrene/butadiene latex from Polymerlatex GmbH),
- 0.75 parts of Walocel CRT 10G as a synthetic co-binder (carboxymethylcellulose from Wolff Cellulosics GmbH & Co KG),
- 0.75 parts of Polyviol LL 603 (polyvinyl alcohol form Wacker-Chemie),
- 0.25 parts Polysalz S as a dispersant based on polyacrylic acid (BASF AG),
- water, and
- 10 % strength sodium hydroxide solution.

The amount of water and the amount of sodium hydroxide solution were chosen to result in a solids content of 60% and a pH of 8.5.

The coating slip was divided into parts and fluorescent whitening agent preparation was added to each part in an amount resulting in 0.15, 0.22, or 0.3% by weight of the fluorescent whitening agent, as indicated in the Tables below, and then stirred for 10 minutes. The amounts added were based on the solids content of the coating slip.

The brightened coating slips obtained were applied by means of a laboratory knife coater (from Erichsen, K-Control Coater, model K202) to wood-free base papers having a basis weight of about 80 g/m². The coated papers were dried for 1 minute at 95°C on a drying cylinder and then stored for 3 hours at 23°C and 50% relative humidity. The measurement of the parameters L*, a*, b* and the determination of the CIE whiteness were then carried out using a whiteness meter (Datacolor Elre-pho SF 450), wherein the light source used was based on ISO 2469 standard.

The following fluorescent whitening agents were used in Examples 1 to 4 and for comparison (Comparative FWA).

### Comparative FWA

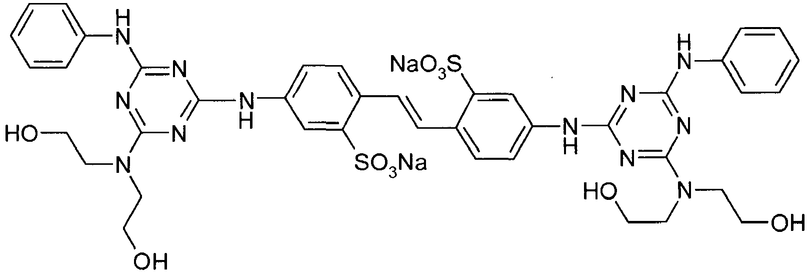

### Example 1

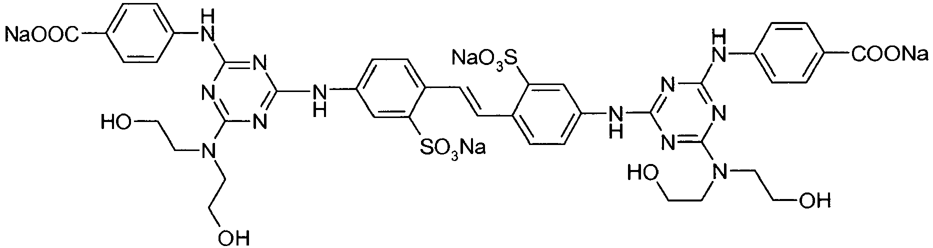

### Example 2

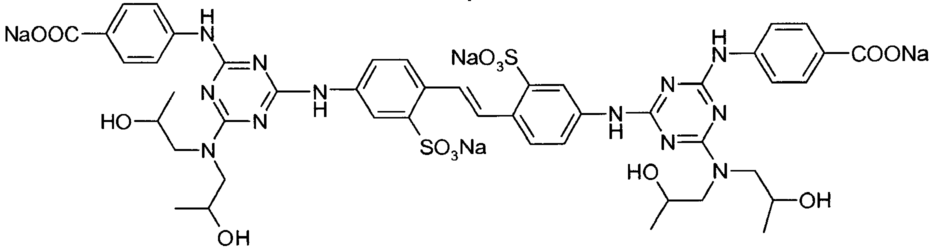

### Example 3

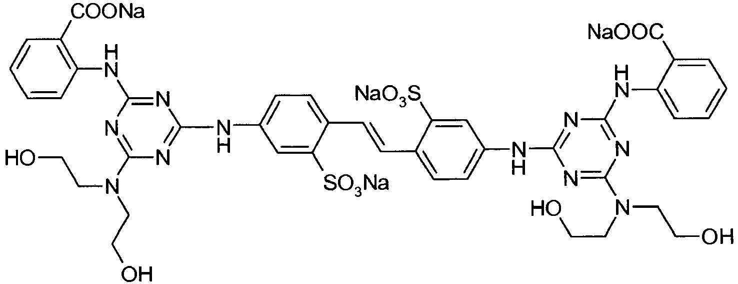

### Example 4

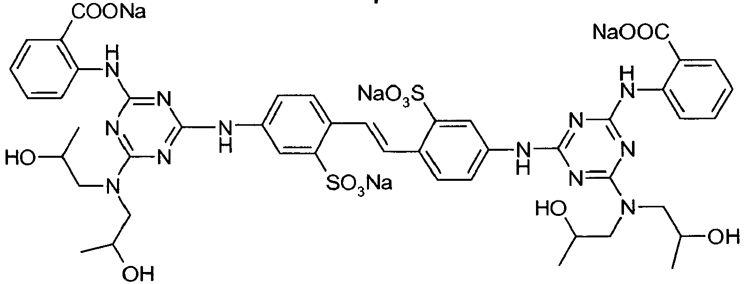

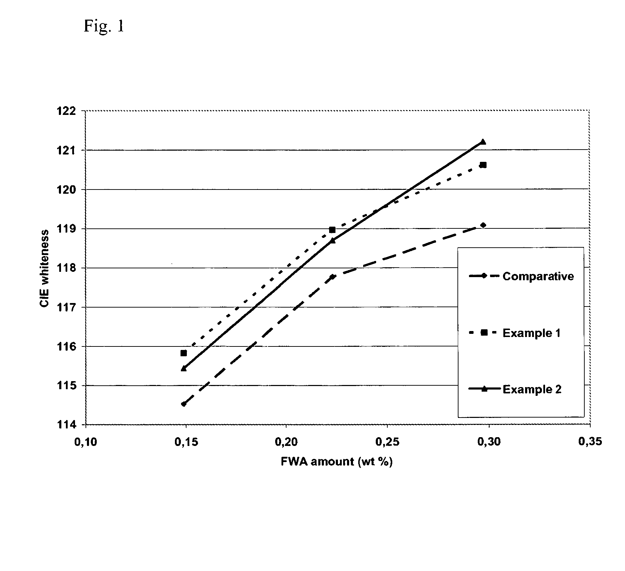

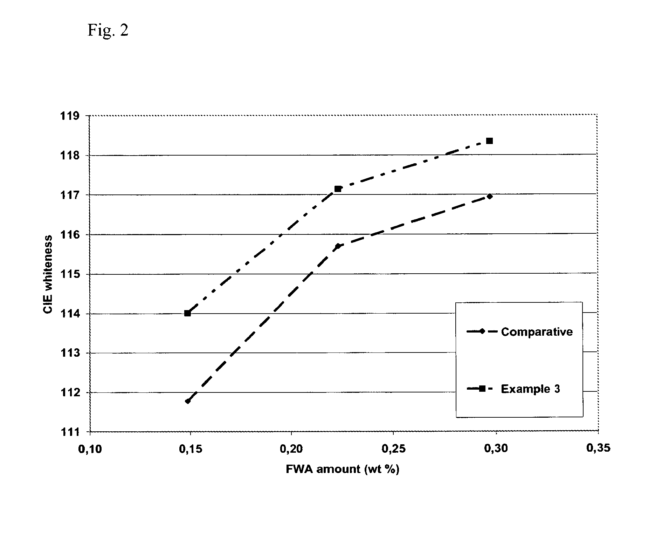

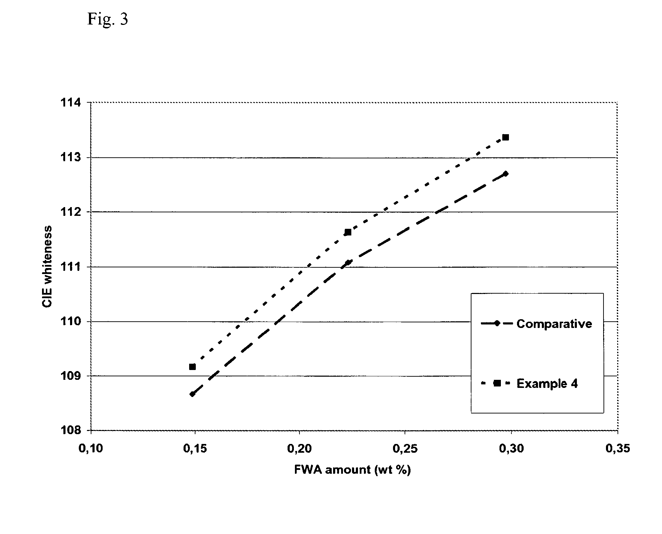

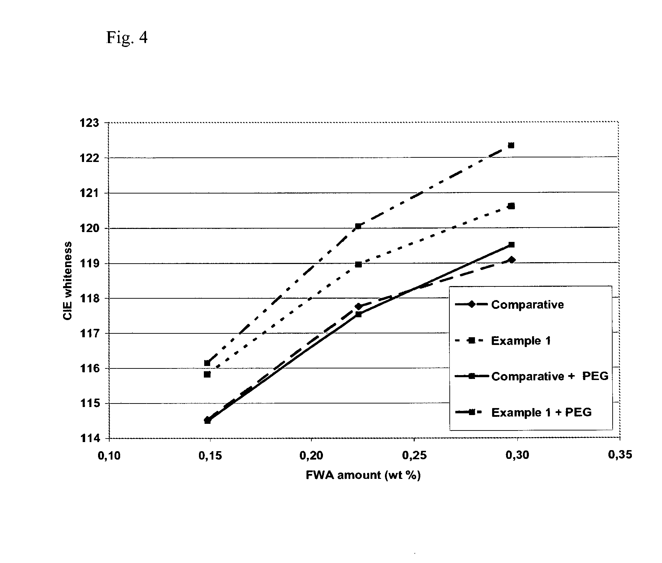

The results obtained are summarized in Tables 1 to 4 and further shown in the corresponding Figures 1 to 4. In all Tables and Figures the same Comparative FWA was used.

**TABLE 1**

| FWA | Amount (wt %) FWA | CIE whiteness | L* | a* | b* |
|---|---|---|---|---|---|
| Example 1 | 0.15 | 115.83 | 95.04 | 1.38 | -6.24 |
| | 0.22 | 118.97 | 95.16 | 1.37 | -6.88 |
| | 0.30 | 120.62 | 95.25 | 1.30 | -7.20 |
| Example 2 | 0.15 | 115.44 | 94.97 | 1.53 | -6.18 |
| | 0.22 | 118.69 | 95.07 | 1.59 | -6.86 |
| | 0.30 | 121.21 | 95.16 | 1.61 | -7.38 |
| Comparative FWA | 0.15 | 114.52 | 94.98 | 1.45 | -5.97 |
| | 0.22 | 117.76 | 95.09 | 1.48 | -6.64 |
| | 0.30 | 119.08 | 95.14 | 1.38 | -6.91 |

**TABLE 2**

| FWA | Amount (wt %) FWA | CIE whiteness | L* | a* | b* |
|---|---|---|---|---|---|
| Example 3 | 0.15 | 114.01 | 95.02 | 1.40 | -5.84 |
| | 0.22 | 117.15 | 95.18 | 1.35 | -6.46 |
| | 0.30 | 118.35 | 95.28 | 1.18 | -6.68 |
| Comparative FWA | 0.15 | 111.77 | 94.90 | 1.48 | -5.40 |
| | 0.22 | 115.70 | 95.09 | 1.51 | -6.18 |
| | 0.30 | 116.94 | 95.16 | 1.38 | -6.43 |

**TABLE 3**

| FWA | Amount (wt %) FWA | CIE whiteness | L* | a* | b* |
|---|---|---|---|---|---|
| Example 4 | 0.15 | 109.17 | 94.73 | 1.28 | -4.90 |
| | 0.22 | 111.64 | 94.80 | 1.26 | -5.42 |
| | 0.30 | 113.37 | 94.85 | 1.23 | -5.77 |
| Comparative FWA | 0.15 | 108.67 | 94.79 | 1.22 | -4.76 |
| | 0.22 | 111.08 | 94.82 | 1.24 | -5.28 |
| | 0.30 | 112.70 | 94.89 | 1.17 | -5.61 |

**TABLE 4**

| FWA | Amount (wt %) FWA | CIE whiteness | L* | a* | b* |
|---|---|---|---|---|---|
| Example 1 | 0.15 | 115.83 | 95.04 | 1.38 | -6.24 |
| | 0.22 | 118.97 | 95.16 | 1.37 | -6.88 |
| | 0.30 | 120.62 | 95.25 | 1.30 | -7.20 |
| Comparative FWA | 0.15 | 114.52 | 94.98 | 1.45 | -5.97 |
| | 0.22 | 117.76 | 95.09 | 1.48 | -6.64 |
| | 0.30 | 119.08 | 95.14 | 1.38 | -6.91 |
| Example 1 + PEG | 0.15 | 116.15 | 95.04 | 1.46 | -6.30 |
| | 0.22 | 120.05 | 95.13 | 1.56 | -7.13 |
| | 0.30 | 122.34 | 95.17 | 1.60 | -7.63 |
| Comparative FWA + PEG | 0.15 | 114.49 | 94.91 | 1.52 | -6.00 |
| | 0.22 | 117.54 | 95.01 | 1.64 | -6.63 |
| | 0.30 | 119.51 | 95.07 | 1.72 | -7.04 |

The results show that the coating slips prepared by using the specific disulfo-type fluorescent whitening agent according to the invention exhibit higher whitening performance compared to the coating slips prepared by using a common disulfotype fluorescent whitening agent (comparative FWA). Furthermore, the addition of polyethylene glycol (PEG) further increases the whitening performance of the coating slips.

## Claims

1. Use of a fluorescent whitening agent of formula (I) 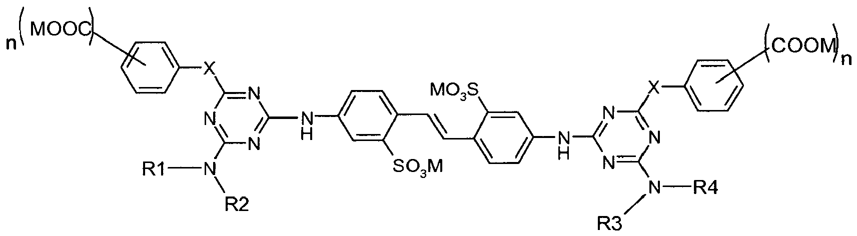 wherein
X represents, independently of each other, O or NR', where R' is hydrogen or C₁-C₃ alkyl;
n is 1 or 2;
R₁, R₂, R₃ and R₄ represent, independently of each other, hydrogen, cyano, C₁ - C₄ alkyl, C₂ - C₄ cyanoalkyl, C₂ - C₄ hydroxyalkyl, or C₁ - C₄ alkoxyalkyl, wherein alkyl is linear or branched; or R₂ and R₁ or R₃ and R₄ independently of each other together with N atom form morpholine, piperidine or pyrrolidine ring; or -(CH₂)₁-SO₃M, where 1 is 1, 2 or 3; or - (CH₂)ᵢ-COOR, -(CH₂)ᵢ-CONHR, -(CH₂)ᵢ-OR, where i is an integer from 1 to 4, R is C₁-C₃ alkyl or has the same meaning as M;
M represents hydrogen, or one equivalent of a cation, in particular Li, Na, K, Ca, Mg, ammonium, or ammonium which is mono-, di-, tri- or tetra-substituted by C₁ - C₄ alkyl or C₂ - C₄ hydroxyalkyl;
for the brightening of coating slips containing at least one synthetic binder and at least one synthetic co-binder differing from the synthetic binder.

2. The use according to claim 1, wherein the fluorescent whitening agent is selected from a compound of formula (Ia) and (Ib) 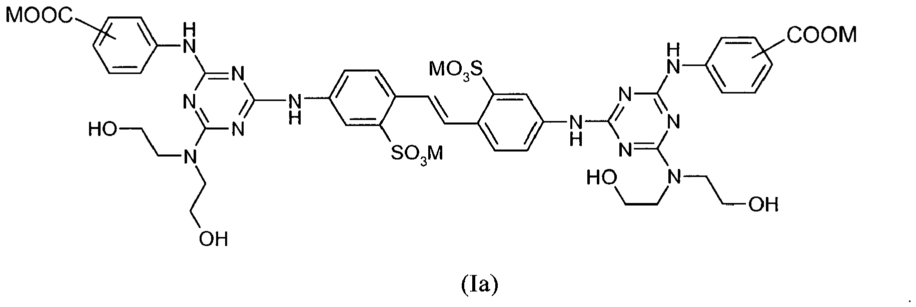 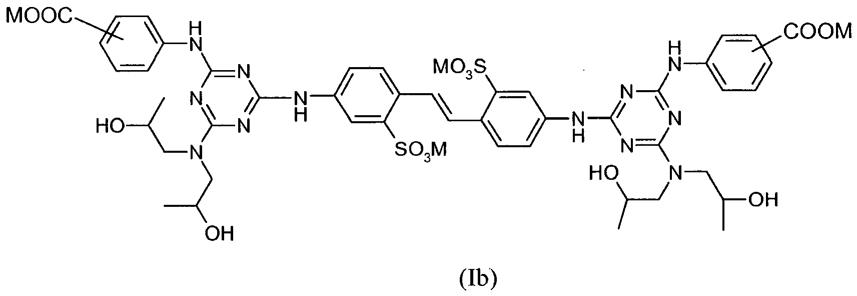 in which M has the meaning defined in claim 1.

3. The use according to claim 1 or 2, wherein X is NR', R' being defined as in claim 1, and R₁, R₂, R₃ and R₄ represent C₂-C₄ hydroxyalkyl.

4. The use according to any of claims 1 to 3, wherein as synthetic binder a latex binder based on styrene/butadiene, styrene/acrylate, or vinyl acetate, is used.

5. The use according to any of the preceding claims, wherein polyethylene glycol, polyvinyl alcohol, carboxymethylcellulose, or a mixture thereof is used as synthetic co-binder.

6. The use according to any of the preceding claims, wherein the coating slip further contains at least one white pigment.

7. The use according to any of the preceding claims, wherein the coating slip contains the synthetic binder in an amount of 3 to 20% by weight, and the co-binder in an amount of 0.1 to 3% by weight, based on 100% by weight of the white pigment of the coating slip.

8. A preparation containing at least fluorescent whitening agent of formula (I) 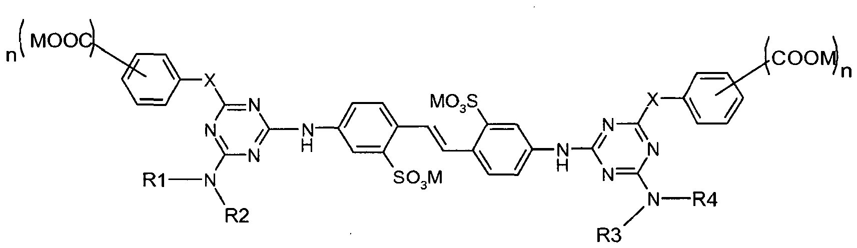 wherein
X represents, independently of each other, O or NR', where R' is hydrogen or C₁-C₃ alkyl;
n is 1 or 2;
R₁, R₂, R₃ and R₄ represent, independently of each other, hydrogen, cyano, C₁ - C₄ alkyl, C₂ - C₄ cyanoalkyl, C₂ - C₄ hydroxyalkyl, or C₁ - C₄ alkoxyalkyl, wherein alkyl is linear or branched; or R₂ and R₁ or R₃ and R₄ independently of each other together with N atom form morpholine, piperidine or pyrrolidine ring; or -(CH₂)₁-SO₃M, where 1 is 1, 2 or 3; or - (CH₂)ᵢ-COOR, -(CH₂)ᵢ-CONHR, -(CH₂)ᵢ-OR, where i is an integer from 1 to 4, R is C₁-C₃ alkyl or has the same meaning as M;
M represents hydrogen, or one equivalent of a cation, in particular Li, Na, K, Ca, Mg, ammonium, or ammonium which is mono-, di-, tri- or tetra-substituted by C₁ - C₄ alkyl or C₂ - C₄ hydroxyalkyl;
and polyethylene glycol.

9. A coating slip containing
- at least one white pigment,
- at least one synthetic binder,
- at least one synthetic co-binder differing from the synthetic binder, and
- at least fluorescent whitening agent of formula (I) 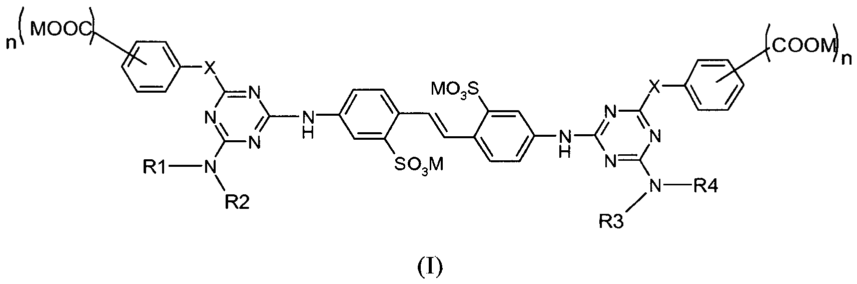 wherein
X represents, independently of each other, O or NR', where R' is hydrogen or C₁-C₃ alkyl;
n is 1 or 2;
R₁, R₂, R₃ and R₄ represent, independently of each other, hydrogen, cyano, C₁ - C₄ alkyl, C₂ - C₄ cyanoalkyl, C₂ - C₄ hydroxyalkyl, or C₁ - C₄ alkoxyalkyl, wherein alkyl is linear or branched; or R₂ and R₁ or R₃ and R₄ independently of each other together with N atom form morpholine, piperidine or pyrrolidine ring; or -(CH₂)₁-SO₃M, where 1 is 1, 2 or 3; or - (CH₂)ᵢ-COOR, -(CH₂)ᵢ-CONHR, -(CH₂)ᵢ-OR, where i is an integer from 1 to 4, R is C₁-C₃ alkyl or has the same meaning as M;
M represents hydrogen, or one equivalent of a cation, in particular Li, Na, K, Ca, Mg, ammonium, or ammonium which is mono-, di-, tri- or tetra-substituted by C₁ - C₄ alkyl or C₂ - C₄ hydroxyalkyl.

10. The coating slip according to claim 9, containing 0.025 to 1% by weight of fluorescent whitening agent of formula (I), 3 to 20% by weight of synthetic binder, and 0.1 to 3% by weight of synthetic co-binder, in each case based on 100% by weight of the white pigment of the coating slip.

11. Use of the coating slip according to claim 9 or 10 for the production of coated papers.

12. Paper obtainable by a process using the coating slip according to claim 9 or 10.

13. A method for brightening a coating slip comprising at least one synthetic binder and at least one synthetic co-binder differing from the synthetic binder, comprising treating the coating slip with a fluorescent whitening agent composition comprising at least one fluorescent whitening agent of formula (I) 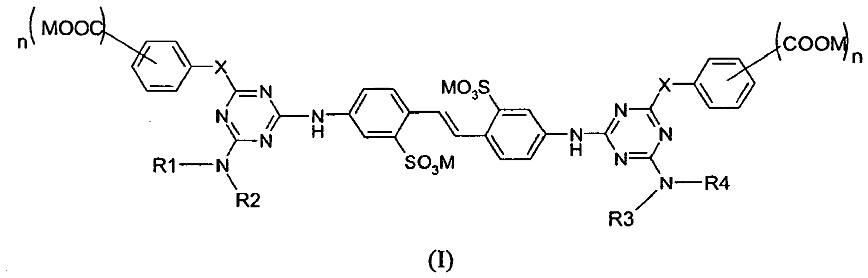 wherein
X represents, independently of each other, O or NR', where R' is hydrogen or C₁-C₃ alkyl;
n is 1 or 2;
R₁, R₂, R₃ and R₄ represent, independently of each other, hydrogen, cyano, C₁ - C₄ alkyl, C₂ - C₄ cyanoalkyl, C₂ - C₄ hydroxyalkyl, or C₁ - C₄ alkoxyalkyl, wherein alkyl is linear or branched; or R₂ and R₁ or R₃ and R₄ independently of each other together with N atom form morpholine, piperidine or pyrrolidine ring; or -(CH₂)₁-SO₃M, where 1 is 1, 2 or 3; or - (CH₂)ᵢ-COOR, -(CH₂)ᵢ-CONHR, -(CH₂)ᵢ-OR, where i is an integer from 1 to 4, R is C₁-C₃ alkyl or has the same meaning as M;
M represents hydrogen, or one equivalent of a cation, in particular Li, Na, K, Ca, Mg, ammonium, or ammonium which is mono-, di-, tri- or tetra-substituted by C₁ - C₄ alkyl or C₂ - C₄ hydroxyalkyl.

14. The method according to claim 13, wherein the at least one synthetic binder is a latex binder based on styrene/butadiene, styrene/acrylate, or vinyl acetate.

15. The method according to claim 13 or 14, wherein the at least one synthetic co-binder is polyethylene glycol, polyvinyl alcohol, carboxymethylcellulose, or a mixture thereof.

## Patentansprüche

1. Verwendung eines fluoreszierenden Weißmachers der Formel (I) 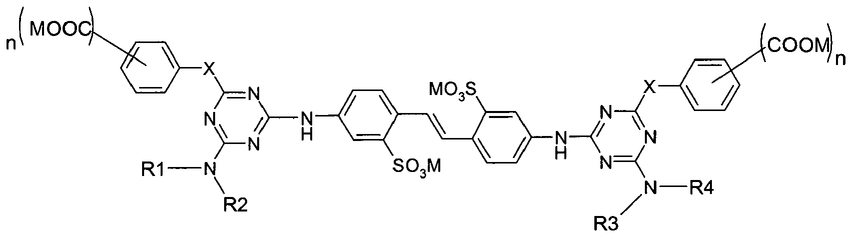 wobei
X unabhängig voneinander O oder NR' darstellt, wobei R' Wasserstoff oder C₁-C₃-Alkyl ist;
n i oder 2 ist;
R₁, R₂, R₃ und R₄ unabhängig voneinander Wasserstoff, Cyano, C₁-C₄-Alkyl, C₂-C₄-Cyanoalkyl, C₂-C₄-Hydroxyalkyl oder C₁-C₄-Alkoxyalkyl darstellen, wobei Alkyl linear oder verzweigt ist; oder R₂ und R₁ oder R₃ und R₄ unabhängig voneinander zusammen mit einem N-Atom einen Morpholin-, Piperidin- oder PyrrolidinRing bilden; oder -(CH₂)ₗ-SO₃M, wobei l 1, 2 oder 3 ist; oder
-(CH₂)ᵢ-COOR, -(CH₂)ᵢ-CONHR, -(CH₂)ᵢ-OR, wobei i eine ganze Zahl von 1 bis 4 ist, R C₁-C₃-Alkyl ist oder die gleiche Bedeutung wie M hat;
M Wasserstoff oder ein Äquivalent eines Kations darstellt, insbesondere Li, Na, K, Ca, Mg, Ammonium oder Ammonium, das mit C₁-C₄-Alkyl oder C₂-C₄-Hydroxyalkyl mono-, di-, tri- oder tetrasubstituiert ist;
zum Aufhellen von Streichmassen, die wenigstens ein synthetisches Bindemittel und wenigstens ein sich von dem synthetischen Bindemittel unterscheidendes, synthetisches Co-Bindemittel enthalten.

2. Verwendung nach Anspruch 1, wobei der fluoreszierende Weißmacher aus einer Verbindung der Formel (Ia) und (Ib) 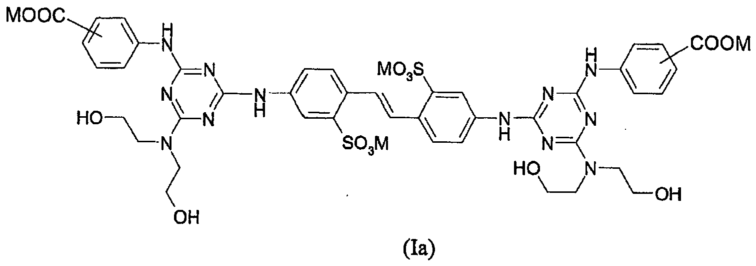 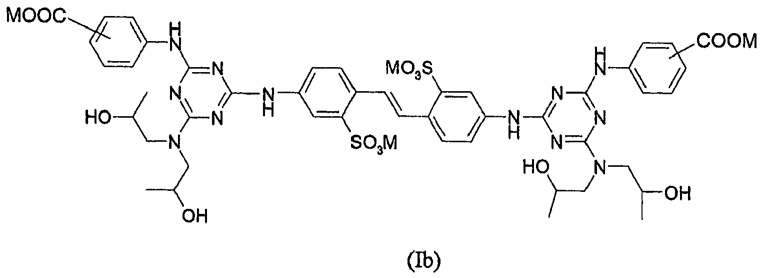 ausgewählt ist, wobei M die in Anspruch 1 definierte Bedeutung hat.

3. Verwendung nach Anspruch 1 oder 2, wobei X NR' ist, R' wie in Anspruch 1 definiert ist und R₁, R₂, R₃ und R₄ C₂-C₄-Hydroxyalkyl darstellen.

4. Verwendung nach irgendeinem der Ansprüche 1 bis 3, wobei als synthetisches Bindemittel ein Latex-Bindemittel auf Basis von Styrol/Butadien, Styrol/Acrylat oder Vinylacetat verwendet wird.

5. Verwendung nach irgendeinem der vorhergehenden Ansprüche, wobei Polyethylenglykol, Polyvinylalkohol, Carboxymethylcellulose oder ein Gemisch davon als synthetisches Co-Bindemittel verwendet wird.

6. Verwendung nach irgendeinem der vorhergehenden Ansprüche, wobei die Streichmasse weiterhin wenigstens ein Weißpigment enthält.

7. Verwendung nach irgendeinem der vorhergehenden Ansprüche, wobei die Streichmasse das synthetische Bindemittel in einer Menge von 3 bis 20 Gewichts-% und das Co-Bindemittel in einer Menge von 0,1 bis 3 Gewichts-%, bezogen auf 100 Gewichts-% des Weißpigments der Streichmasse, enthält.

8. Präparation, enthaltend wenigstens einen fluoreszierenden Weißmacher der Formel (I) 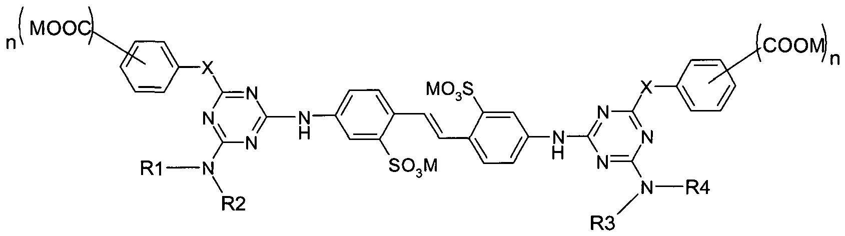 wobei
X unabhängig voneinander O oder NR' darstellt, wobei R' Wasserstoff oder C₁-C₃-Alkyl ist;
n i oder 2 ist;
R₁, R₂, R₃ und R₄ unabhängig voneinander Wasserstoff, Cyano, C₁-C₄-Alkyl, C₂-C₄-Cyanoalkyl, C₂-C₄-Hydroxyalkyl oder C₁-C₄Alkoxyalkyl darstellen, wobei Alkyl linear oder verzweigt ist; oder R₂ und R₁ oder R₃ und R₄ unabhängig voneinander zusammen mit einem N-Atom einen Morpholin-, Piperidin- oder PyrrolidinRing bilden; oder -(CH₂)ₗ-SO₃M, wobei l 1, 2 oder 3 ist; oder
-(CH₂)ᵢ-COOR, -(CH₂)ᵢ-CONHR, -(CH₂)ᵢ-OR, wobei i eine ganze Zahl von 1 bis 4 ist, R C₁-C₃-Alkyl ist oder die gleiche Bedeutung wie M hat;
M Wasserstoff oder ein Äquivalent eines Kations darstellt, insbesondere Li, Na, K, Ca, Mg, Ammonium oder Ammonium, das mit C₁-C₄-Alkyl oder C₂-C₄-Hydroxyalkyl mono-, di-, tri- oder tetrasubstituiert ist;
und Polyethylenglykol.

9. Streichmasse, enthaltend:
- wenigstens ein Weißpigment,
- wenigstens ein synthetisches Bindemittel,
- wenigstens ein sich von dem synthetischen Bindemittel unterscheidendes, synthetisches Co-Bindemittel, und
- wenigstens einen fluoreszierenden Weißmacher der Formel (I) 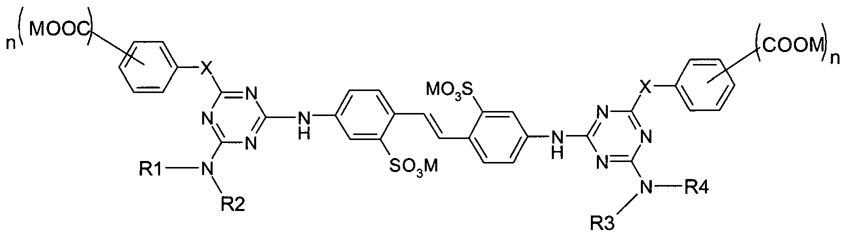 wobei
X unabhängig voneinander O oder NR' darstellt, wobei R' Wasserstoff oder C₁-C₃-Alkyl ist;
n 1 oder 2 ist;
R₁, R₂, R₃ und R₄ unabhängig voneinander Wasserstoff, Cyano, C₁-C₄-Alkyl, C₂-C₄-Cyanoalkyl, C₂-C₄-Hydroxyalkyl oder C₁-C₄-Alkoxyalkyl darstellen, wobei Alkyl linear oder verzweigt ist; oder R₂ und R₁ oder R₃ und R₄ unabhängig voneinander zusammen mit einem N-Atom einen Morpholin-, Piperidin- oder PyrrolidinRing bilden; oder -(CH₂)ₗ-SO₃M, wobei l 1, 2 oder 3 ist; oder
-(CH₂)ᵢ-COOR, -(CH₂)ᵢ-CONHR, -(CH₂)ᵢ-OR, wobei i eine ganze Zahl von 1 bis 4 ist, R C₁-C₃-Alkyl ist oder die gleiche Bedeutung wie M hat;
M Wasserstoff oder ein Äquivalent eines Kations darstellt, insbesondere Li, Na, K, Ca, Mg, Ammonium oder Ammonium, das mit C₁-C₄-Alkyl oder C₂-C₄-Hydroxyalkyl mono-, di-, tri- oder tetrasubstituiert ist.

10. Streichmasse nach Anspruch 9, enthaltend 0,025 bis 1 Gewichts-% des fluoreszierenden Weißmachers der Formel (I), 3 bis 20 Gewichts-% synthetisches Bindemittel und 0,1 bis 3 Gewichts-% synthetisches Co-Bindemittel, jeweils bezogen auf 100 Gewichts-% des Weißpigments der Streichmasse.

11. Verwendung der Streichmasse nach Anspruch 9 oder 10 zur Herstellung von gestrichenen Papieren.

12. Papier, erhältlich durch ein Verfahren unter Verwendung der Streichmasse nach Anspruch 9 oder 10.

13. Verfahren zum Aufhellen einer Streichmasse, umfassend wenigstens ein synthetisches Bindemittel und wenigstens ein sich von dem synthetischen Bindemittel unterscheidendes, synthetisches Co-Bindemittel, umfassend das Behandeln der Streichmasse mit einer fluoreszierenden Weißmacher-Zusammensetzung, umfassend wenigstens einen fluoreszierenden Weißmacher der Formel (I) 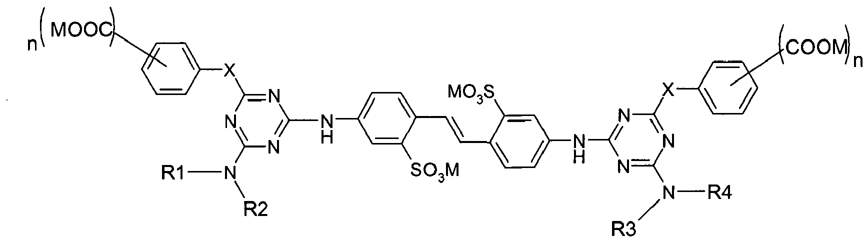 wobei
X unabhängig voneinander O oder NR' darstellt, wobei R' Wasserstoff oder C₁-C₃-Alkyl ist;
n 1 oder 2 ist;
R₁, R₂, R₃ und R₄ unabhängig voneinander Wasserstoff, Cyano, C₁-C₄-Alkyl, C₂-C₄-Cyanoalkyl, C₂-C₄-Hydroxyalkyl oder C₁-C₄-Alkoxyalkyl darstellen, wobei Alkyl linear oder verzweigt ist; oder R₂ und R₁ oder R₃ und R₄ unabhängig voneinander zusammen mit einem N-Atom einen Morpholin-, Piperidin- oder PyrrolidinRing bilden; oder -(CH₂)ₗ-SO₃M, wobei l 1, 2 oder 3 ist; oder
-(CH₂)ᵢ-COOR, -(CH₂)ᵢ-CONHR, -(CH₂)ᵢ-OR, wobei i eine ganze Zahl von 1 bis 4 ist, R C₁-C₃-Alkyl ist oder die gleiche Bedeutung wie M hat;
M Wasserstoff oder ein Äquivalent eines Kations darstellt, insbesondere Li, Na, K, Ca, Mg, Ammonium oder Ammonium, das mit C₁-C₄-Alkyl oder C₂-C₄-Hydroxyalkyl mono-, di-, tri- oder tetrasubstituiert ist.

14. Verfahren nach Anspruch 13, wobei das mindestens eine synthetische Bindemittel ein Latex-Bindemittel auf Basis von Styrol/Butadien, Styrol/Acrylat oder Vinylacetat ist.

15. Verfahren nach Anspruch 13 oder 14, wobei das mindestens eine synthetische Co-Bindemittel Polyethylenglykol, Polyvinylalkohol, Carboxymethylcellulose oder ein Gemisch davon ist.

## Revendications

1. Utilisation d'un azurant fluorescent de formule (I) 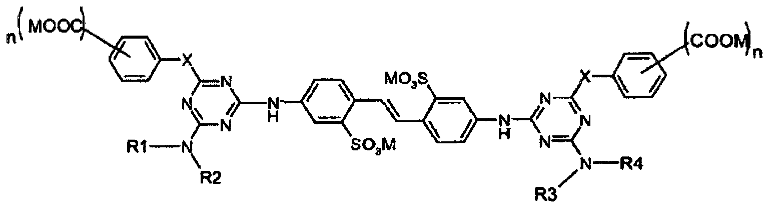 dans laquelle
les radicaux X représentent, chacun indépendamment de l'autre, O ou NR', R' étant un atome d'hydrogène ou un groupe alkyle en C₁-C₃ ;
n vaut 1 ou 2 ;
R₁, R₂, R₃ et R₄ représentent chacun indépendamment des autres un atome d'hydrogène, un groupe cyano, alkyle en C₁-C₄, cyanoalkyle en C₂-C₄, hydroxyalkyle en C₂-C₄ ou alcoxyalkyle en C₁-C₄, où le fragment alkyle est à chaîne droite ou ramifiée ; ou R₂ et R₁ ou R₃ et R₄, forment d'une manière indépendante, avec l'atome d'azote, un noyau morpholine, pipéridine ou pyrrolidine ; ou -(CH₂)ₗ-SO₃M_{,} où l vaut 1, 2 ou 3 ; ou -(CH₂)ᵢ-COOR, -(CH2) i - CONHR, -(CH₂)ᵢ-OR, où i est un entier de 1 à 4, R est un groupe alkyle en C₁-C₃ ou a les mêmes significations que M ;
M représente un atome d'hydrogène ou un équivalent d'un cation, en particulier Li, Na, K, Ca, Mg, ammonium, ou ammonium qui est mono-, di-, tri- ou tétra-substitué par un ou plusieurs substituants alkyle en C₁-C₄ ou hydroxyalkyle en C₂-C₄ ;
pour le blanchiment de sauces de couchage contenant au moins un liant synthétique et au moins un co-liant synthétique différent du liant synthétique.

2. Utilisation selon la revendication 1, pour laquelle l'azurant fluorescent est choisi parmi les composés de formule (Ia) et (Ib) 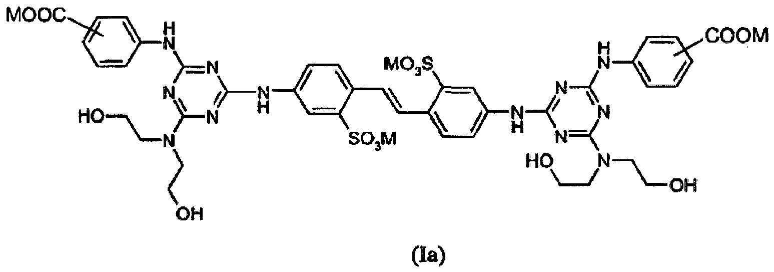 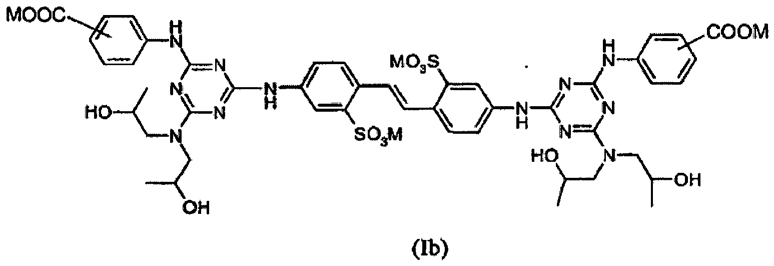 où M a les significations données dans la revendication 1.

3. Utilisation selon la revendication 1 ou 2, pour laquelle X est NR', R' étant défini comme dans la revendication 1, et R₁, R₂, R₃ et R₄ représentent des groupes hydroxyalkyle en C₂-C₄.

4. Utilisation selon l'une quelconque des revendications 1 à 3, pour laquelle on utilise en tant que liant synthétique un liant de type latex à base de styrène/butadiène, de styrène/acrylate ou d'acétate de vinyle.

5. Utilisation selon l'une quelconque des revendications précédentes, pour laquelle on utilise en tant que co-liant synthétique du polyéthylèneglycol, du poly(alcool vinylique), de la carboxyméthylcellulose ou un mélange de ceux-ci.

6. Utilisation selon l'une quelconque des revendications précédentes, pour laquelle la sauce de couchage contient en outre au moins un pigment blanc.

7. Utilisation selon l'une quelconque des revendications précédentes, pour laquelle la sauce de couchage contient le liant synthétique en une quantité de 3 à 20 % en poids, et le co-liant en une quantité de 0,1 à 3 % en poids, pour 100 % en poids du pigment blanc de la sauce de couchage.

8. Préparation contenant au moins un azurant fluorescent de formule (I) 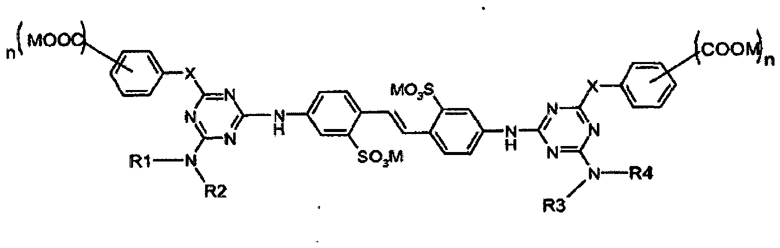 dans laquelle
les radicaux X représentent, chacun indépendamment de l'autre, O ou NR', R' étant un atome d'hydrogène ou un groupe alkyle en C₁-C₃ ;
n vaut 1 ou 2 ;
R₁, R₂, R₃ et R₄ représentent chacun indépendamment des autres un atome d'hydrogène, un groupe cyano, alkyle en C₁-C₄, cyanoalkyle en C₂-C₄, hydroxyalkyle en C₂-C₄ ou alcoxyalkyle en C₁-C₄, où le fragment alkyle est à chaîne droite ou ramifiée ; ou R₂ et R₁ ou R₃ et R₄, forment d'une manière indépendante, avec l'atome d'azote, un noyau morpholine, pipéridine ou pyrrolidine ; ou -(CH₂)₁-SO₃M, où 1 vaut 1, 2 ou 3 ; ou -(CH₂)ᵢ-COOR, - (CH₂)ᵢ-CONHR, -(CH₂)ᵢ-OR, où i est un entier de 1 à 4, R est un groupe alkyle en C₁-C₃ ou a les mêmes significations que M ;
M représente un atome d'hydrogène ou un équivalent d'un cation, en particulier Li, Na, K, Ca, Mg, ammonium, ou ammonium qui est mono-, di-, tri- ou tétra-substitué par un ou plusieurs substituants alkyle en C₁-C₄ ou hydroxyalkyle en C₂-C₄ ;
et du polyéthylèneglycol.

9. Sauce de couchage contenant
- au moins un pigment blanc,
- au moins un liant synthétique,
- au moins un co-liant synthétique différent du liant synthétique, et
- au moins un azurant fluorescent de formule (I) 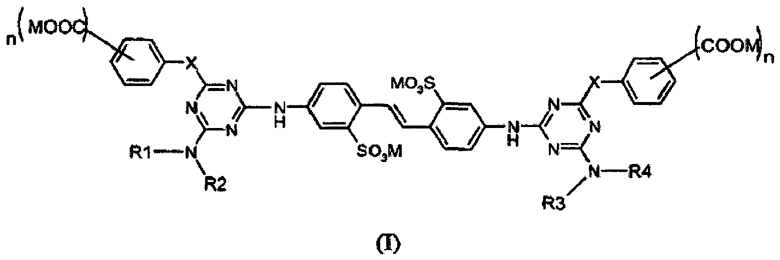 dans laquelle
les radicaux X représentent, chacun indépendamment de l'autre, O ou NR', R' étant un atome d'hydrogène ou un groupe alkyle en C₁-C₃ ;
n vaut 1 ou 2 ;
R₁, R₂, R₃ et R₄ représentent chacun indépendamment des autres un atome d'hydrogène, un groupe cyano, alkyle en C₁-C₄, cyanoalkyle en C₂-C₄, hydroxyalkyle en C₂-C₄ ou alcoxyalkyle en C₁-C₄, où le fragment alkyle est à chaîne droite ou ramifiée ; ou R₂ et R₁ ou R₃ et R₄, forment d'une manière indépendante, avec l'atome d'azote, un noyau morpholine, pipéridine ou pyrrolidine ; ou -(CH₂)ₗ-SO₃M, où 1 vaut 1, 2 ou 3 ; ou -(CH₂)ᵢ-COOR, -(CH₂)ᵢ-CONHR , -(CH₂)ᵢ-OR, où i est un entier de 1 à 4, R est un groupe alkyle en C₁-C₃ ou a les mêmes significations que M ;
M représente un atome d'hydrogène ou un équivalent d'un cation, en particulier Li, Na, K, Ca, Mg, ammonium, ou ammonium qui est mono-, di-, tri- ou tétra-substitué par un ou plusieurs substituants alkyle en C₁-C₄ ou hydroxyalkyle en C₂-C₄.

10. Sauce de couchage selon la revendication 9, contenant 0,025 à 1 % en poids de l'azurant fluorescent de formule (I), 3 à 20 % en poids d'un liant synthétique, et 0,1 à 3 % en poids d'un co-liant synthétique, dans chaque cas pour 100 % en poids du pigment blanc de la sauce de couchage.

11. Utilisation de la sauce de couchage selon la revendication 9 ou 10 pour la production de papiers couchés.

12. Papier pouvant être obtenu par un procédé utilisant la sauce de couchage selon la revendication 9 ou 10.

13. Procédé de blanchiment d'une sauce de couchage comprenant au moins un liant synthétique et au moins un co-liant synthétique différent du liant synthétique, comprenant le traitement de la sauce de couchage avec une composition d'azurant fluorescent comprenant au moins un azurant fluorescent de formule (I) 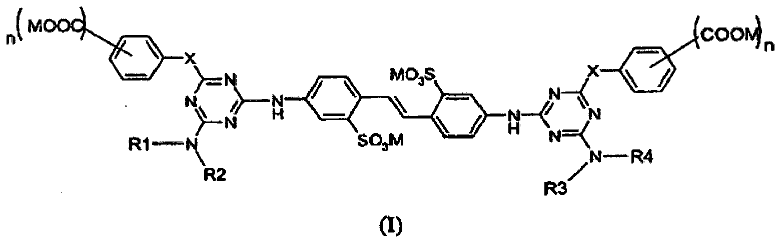 dans laquelle
les radicaux X représentent, chacun indépendamment de l'autre, O ou NR', R' étant un atome d'hydrogène ou un groupe alkyle en C₁-C₃ ;
n vaut 1 ou 2 ;
R₁, R₂, R₃ et R₄ représentent chacun indépendamment des autres un atome d'hydrogène, un groupe cyano, alkyle en C₁-C₄, cyanoalkyle en C₂-C₄, hydroxyalkyle en C₂-C₄ ou alcoxyalkyle en C₁-C₄, où le fragment alkyle est à chaîne droite ou ramifiée ; ou R₂ et R₁ ou R₃ et R₄, forment d'une manière indépendante, avec l'atome d'azote, un noyau morpholine, pipéridine ou pyrrolidine ; ou -(CH₂)₁-SO₃M, où l vaut 1, 2 ou 3 ; ou -(CH₂)ᵢ-COOR, -(CH₂)ᵢ-CONHR, -(CH₂)ᵢ-OR, où i est un entier de 1 à 4, R est un groupe alkyle en C₁-C₃ ou a les mêmes significations que M ;
M représente un atome d'hydrogène ou un équivalent d'un cation, en particulier Li, Na, K, Ca, Mg, ammonium, ou ammonium qui est mono-, di-, tri- ou tétra-substitué par un ou plusieurs substituants alkyle en C₁-C₄ ou hydroxyalkyle en C₂-C₄.

14. Procédé selon la revendication 13, dans lequel l'au moins un liant synthétique est un liant de type latex à base de styrène/butadiène, de styrène/acrylate ou d'acétate de vinyle.

15. Procédé selon la revendication 13 ou 14, dans lequel l'au moins un co-liant synthétique est le polyéthylèneglycol, le poly(alcool vinylique), la carboxyméthylcellulose ou un mélange de ceux-ci.
